# DEMANDE DE BREVET EUROPEEN

(11) **EP 1 808 159 A1**
(43) Date de publication de la demande: **18.07.2007**
(21) Numéro de dépôt: 06291992.3
(22) Date de dépôt: 20.12.2006
(51) Int. Cl.: A61K 9/00, A61K 36/53, A61P 11/02, A61P 11/04

(54) **Nouvelle composition pharmaceutique à base d'huile essentielle pour pulverisation nasale et/ou buccale**

(30) Priorité: 23.12.2005 FR 0513257
(71) Demandeur: Les Laboratoires Servier, 92415 Courbevoie Cedex (FR)
(72) Inventeur: Marsas, Stephanie, 45430 Checy (FR); Agasse, Jerome, 45130 Saint Ay (FR); Duburque, Elisabeth, 45000 Orleans (FR); Montes, Pierre, 92700 Colombes (FR)

(57) **Abrégé**

L'invention concerne l'utilisation d'une nouvelle composition pharmaceutique sous forme d'aérosol contenant de l'huile essentielle de menthe poivrée destinée à être utilisée dans le traitement des états congestifs des voies aériennes supérieures, en cas de nez bouché, de rhume, et comme antalgique dans les affections de la cavité buccale et /ou du pharynx.

## Description

La présente invention se rapporte à une nouvelle composition pharmaceutique à base d'huile essentielle pour pulvérisation nasale et/ou buccale, son procédé de préparation et son utilisation à des fins thérapeutiques.

Cette nouvelle composition se présente sous forme d'aérosol, contenant une huile essentielle, plus spécifiquement l'huile essentielle de menthe poivrée. Cette composition pharmaceutique présente un effet décongestionnant nasal, débouche le nez, qui s'accompagne d'une sensation d'amélioration de la respiration, et d'un effet antalgique au niveau de la gorge, soulage les irritations et les maux de gorge.

L'utilisation d'un aérosol pharmaceutique administrée par voie nasale et oro-pharyngée est déjà connu. Or, cet aérosol pharmaceutique peut présenter quelques inconvénients liés à un antibiotique.

Une alternative à l'utilisation d'un antibiotique dans un aérosol thérapeutique pour l'administration par voie orale et nasale est l'utilisation d'une huile essentielle, l'huile essentielle de menthe poivrée, dans un aérosol thérapeutique.

La menthe poivrée, encore appelée mentha piperita, est bien connue pour ses propriétés médicinales à vertu digestive. Le Carominthe® est un médicament contenant de la menthe poivrée connu pour son utilisation pour les troubles fonctionnels digestifs. Elle tonifie également le système nerveux. La menthe poivrée peut également agir sur les maux de tête et migraines. Elle a une action antalgique "secondaire" car, appliquée sur la peau, elle provoque une intense sensation de froid qui calme la douleur pour un temps. De plus, le menthol induit aussi un blocage voltage-dépendant des canaux sodiques, ce qui explique son effet antalgique local.

D'autres propriétés de la menthe poivrée sont encore connues à savoir : propriétés stimulantes, anti-infectieuse, bactéricide, anti-dépresseur, anti-spasmodique et, est efficace contre les jambes lourdes. Par conséquent, les indications connues sont les suivantes : insuffisance hépatopancréatique, vomissements, mal de transports, asthénie, migraines, céphalées, névralgie, sciatique, angoisse, fatigue et lassitude. Le Poconeol^{®} est un médicament connu en tant que sédatif cardiaque.

L'huile essentielle de menthe poivrée possède encore des propriétés décongestionnantes et s'avère efficace contre le rhume et la grippe. En effet, le menthol active à faibles doses les récepteurs nerveux sensibles au froid présents dans le vestibule nasal et au niveau des voies aériennes supérieures, procurant ainsi une sensation de fraîcheur et d'amélioration de la respiration, et une décongestion.

Cependant, lorsqu'elle est utilisée pour ses propriétés décongestionnantes et antalgiques, la menthe poivrée existe soit sous forme de sachet, par exemple en tisane, soit en solution buvable ou encore sous forme de préparation pour inhalations.

Le but de la présente invention consiste en l'élaboration d'une nouvelle formulation en flacon pressurisé, présentant des propriétés décongestionnantes et antalgiques, à base d'huile essentielle de menthe poivrée encore appelée mentha piperita.

De part les indications dans laquelle elle est utilisée, l'huile essentielle de menthe poivrée est essentiellement administrée par voie orale (buccale) ou nasale.

Ainsi, la composition pharmaceutique selon l'invention se présente sous forme d'un aérosol dont la formule renferme, outre le principe actif, l'huile essentielle de menthe poivrée, un mélange d'excipients ainsi qu'un gaz propulseur liquéfié indispensable à tout aérosol, un arôme et un édulcorant.

Cette composition pharmaceutique présente la caractéristique particulièrement avantageuse d'être une solution de principe actif contenant un nombre très limité d'excipients à pouvoir solvant tels que l'éthanol, le myristate d'isopropyle ou le diméthylisosorbide.

Un autre avantage, résultant également du fait que cet aérosol est une solution, est que la fabrication d'une telle formulation est facilitée : les phénomènes de démixion, floculation, sédimentation, manque d'homogénéité rencontrés avec les suspensions n'existent plus.

L'invention concerne donc l'utilisation d'une nouvelle composition pharmaceutique à base d'huile essentielle de menthe poivrée destinée à être utilisée dans le traitement des états congestifs des voies aériennes supérieures, en cas de nez bouché, de rhume, et comme antalgique dans les affections de la cavité buccale et /ou du pharynx.

L'invention concerne également les compositions pharmaceutiques sous forme d'aérosol pressurisé contenant de l'huile essentielle de menthe poivrée en combinaison avec un ou plusieurs excipients pharmaceutiquement acceptables.

Parmi les compositions pharmaceutiques selon l'invention, on pourra citer plus particulièrement celles qui conviennent pour l'administration buccale et nasale.

Selon une variante de l'invention, les compositions pharmaceutiques peuvent renfermer, outre le principe actif, un mélange d'excipients et un gaz propulseur liquéfié, des arômes et des édulcorants.

Par composition aromatique 52931, on entend un mélange des essences suivantes : coriandre, badiane, piment baies et romarin ainsi que la vanilline.

Préférentiellement, les excipients utilisés dans le cadre de l'invention sont des excipients à pouvoir solvant tels que l'éthanol et/ou le myristate d'isopropyle.

Parmi les différents gaz propulseurs, ceux utilisés préférentiellement dans le cadre de l'invention sont les gaz propulseurs liquéfiés sont : 1,1,1,2-tétrafluoroéthane (HFA134a), 1,1,1,2,3,3,3-heptafluoropropane (HFA227) et/ou difluoroéthane (HFA152a).

Plus préférentiellement, le gaz propulseur liquéfié utilisé dans le cadre de l'invention est le 1,1,1,2-tétrafluoroéthane (HFA134a) encore appelé le norflurane.

Parmi les édulcorants, on peut citer à titre non limitatif, la saccharine et l'aspartame.

La présente invention s'étend également au procédé de préparation d'une composition pharmaceutique qui consiste à mélanger le principe actif, le mélange d'excipients et la composition aromatique et enfin à dissoudre l'édulcorant puis à remplir un récipient d'aérosol avec la composition pharmaceutique telle que préparée précédemment, à équiper le récipient d'aérosol d'une valve distributrice doseuse puis à rajouter le gaz propulseur liquéfié.

De façon générale, la nouvelle forme pharmaceutique peut être présentée dans des bidons dont la taille varie entre 5 et 100 ml.

Le principe actif, l'huile essentielle de menthe poivrée, entre dans des proportions de 0,1 à 1% en poids et plus préférentiellement entre 0,1 et 0,5% en poids de la formulation selon l'invention.

Le gaz propulseur liquéfié entre dans une proportion comprise entre 50 et 95% en poids, préférentiellement entre 70 et 90% en poids de la formulation.

L'huile essentielle de menthe poivrée est incorporée à un mélange d'excipients choisi parmi diméthylsosorbide et préférentiellement myristate d'isopropyle, mélange d'excipients qui entre dans la composition dans une proportion comprise entre 1 et 25% en poids, préférentiellement entre 10 et 20% en poids de la formulation.

L'arôme entre dans une proportion comprise entre 0,1 et 5% en poids, préférentiellement entre 0,5 et 2% en poids de la formulation.

L'édulcorant entre dans une proportion comprise entre 0,0005 et 0,1% en poids, préférentiellement entre 0,001 et 0,01 % en poids de la formulation.

L'éthanol anhydre entre préférentiellement dans une proportion comprise entre 0,5 et 5% en poids, préférentiellement entre 0,5 et 2% en poids de la formulation.

La posologie utile varie selon l'âge et le poids du patient, la voie d'administration, la nature de l'affection et des traitements éventuellement associés et s'échelonne entre 0,10 mg et 10,00 mg d'huile essentielle de menthe poivrée par 24 heures et plus préférentiellement 0,50 mg et 5,00 mg par 24 heures.

### EXEMPLE 1 : Composition pharmaceutique

Composition pour un flacon de 8 ml :

| | |
|---|---|
| Huile essentielle de menthe poivrée | 20 mg |
| Composition aromatique 52931 | 60 mg |
| Ethanol anhydre | 96 mg |
| Saccharine | 0,5 mg |
| Myristate d'isopropyle | 1504 mg |
| Norflurane (1,1,1,2-tétrafluoroéthane) | 7260 mg |

La formulation précédente est préparée par mélange des différents constituants et agitation. La quantité requise du mélange est introduite dans un flacon adéquat et une valve distributrice doseuse pour aérosol est sertit sur le flacon. Le propulseur 134a est incorporé sous pression.

### EXEMPLE 2 : Composition pharmaceutique

Composition pour un flacon de 8 ml :

| | |
|---|---|
| Huile essentielle de menthe poivrée | 25 mg |
| Composition aromatique 52931 | 40 mg |
| Ethanol anhydre | 96 mg |
| Saccharine | 0,25 mg |
| Myristate d'isopropyle | 1504 mg |
| Norflurane (1,1,1,2-tétrafluoroéthane) | 7320 mg |

### EXEMPLE 3 : Composition pharmaceutique

Composition pour un flacon de 8 ml :

| | |
|---|---|
| Huile essentielle de menthe poivrée | 40 mg |
| Composition aromatique 52931 | 80 mg |
| Ethanol anhydre | 96 mg |
| Saccharine | 0,5 mg |
| Myristate d'isopropyle | 1504 mg |
| Norflurane (1,1,1,2-tétrafluoroéthane) | 7230 mg |

### EXEMPLE 4 : Composition pharmaceutique

Composition pour un flacon de 8 ml :

| | |
|---|---|
| Huile essentielle de menthe poivrée | 8 mg |
| Composition aromatique 52931 | 80 mg |
| Ethanol anhydre | 96 mg |
| Saccharine | 0,5 mg |
| Myristate d'isopropyle | 1504 mg |
| Norflurane (1,1,1,2-tétrafluoroéthane) | 7280 mg |

### Données cliniques : "Evaluation de l'acceptabilité de l'exemple 1 : étude multicentrique, non comparative, en ouvert, de phase II, chez des sujets présentant une rhinopharyngite aiguë".

L'étude a été réalisée chez 53 sujets présentant une rhinopharyngite aiguë.
L'objectif primaire a été d'évaluer l'acceptabilité de la composition pharmaceutique de l'exemple 1, administré localement en 4 administrations par jour, chez des sujets présentant une rhinopharyngite aiguë. L'acceptabilité a été évaluée d'après le carnet patient et a été évaluée par examen de la rhinopharyngite par l'investigateur avant et après la période de traitement.

### Traitements administrés

La composition pharmaceutique de l'exemple 1 a été administré par voie nasale et orale en 4 administrations par jour, chaque administration consistant en 4 pulvérisations dans la gorge et 2 pulvérisations dans chaque narine, pendant 10 jours (320 pulvérisations sur 10 jours). Chaque pulvérisation contenait 50 µl de solution, c'est-à-dire 0,125 mg d'huile essentielle de menthe poivrée par pulvérisation ou 4 mg par jour (32 pulvérisations).
Visite de suivi (J5)
Evolution de la rhinopharyngite

### Durée de traitement

La durée minimale requise selon le protocole était de 5 jours et la durée théorique de l'étude était de 10 jours, avec les périodes suivantes: visite de sélection/d'inclusion (Jour 1), période de traitement pendant 10 jours (du Jour 1 jusqu'au Jour 10), visite de suivi (Jour 5) et visite de fin d'étude (Jour 10). Seulement les sujets ayant au moins 5 jours d'administration ont été évalués.
Les sujets ont rendu compte, en somme, de la bonne acceptabilité du produit, la plupart d'entre eux considérant comme suffisants le nombre testé d'administrations par jour (4) et le nombre testé de pulvérisations par administration (8).

### Examen du rhinopharynx

**Tableau 1-Evaluation de symptômes dans le groupe de tolérance (N=53)**

| | Tous (N=53) | | | | | |
|---|---|---|---|---|---|---|
| | Triage (N=53) | | Jour 5 (N=53) | | Jour 10 (N=42) | |
| | Présent | Absent | Présent | Absent | Présent | Absent |
| Inflammation des voies supérieures | 49 (92,5) | 4 (7,5) | 31 (58,5) | 22 (41,5) | 6 (14,3) | 36 (85,7) |
| Dysphagie | 39 (73,6) | 14 (26,4) | 16 (30,2) | 37 (69,8) | 4 (9,5) | 38 (90,5) |
| Rhinorrhée | 51 (96,2) | 2 (3,8) | 31 (58,5) | 22 (41,5) | 7 (16,7) | 35 (83,3) |
| Obstruction nasale (nez bouché) | 42 (79,2) | 11 (20,8) | 15 (28,3) | 38 (71,7) | 4 (9,5) | 38 (90,5) |
| Sécrétion nasale (nez qui coule) | 49 (92,5) | 4 (7,5) | 32 (60,4) | 21 (39,6) | 13 (31,0) | 29 (69,0) |
| Erythème de la muqueuse nasale | 48 (90,6) | 5 (9,4) | 24 (45,3) | 29 (54,7) | 6 (14,3) | 36 (85,7) |
| Oedème de la muqueuse nasale | 24 (45,3) | 29 (54,7) | 9 (17,0) | 44 (83,0) | 1 (2,4) | 41 (97,6) |
| Eternuements | 28 (52,8) | 25 (47,2) | 6 (11,3) | 47 (88,7) | 4 (9,5) | 38 (90,5) |
| Toux | 32 (60,4) | 21 (39,6) | 21 (39,6) | 32 (60,4) | 10 (23,8) | 32 (76,2) |
| Ecoulement aqueux | 22 (41,5) | 31 (58,5) | 4 (7,5) | 49 (92,5) | 1 (2,4) | 41 (97,6) |
| Mal de tête | 30 (56,6) | 23 (43,4) | 8 (15,1) | 45 (84,9) | 1 (2,4) | 41 (97,6) |
| Fièvre | 27 (50,9) | 26 (49,1) | 4 (7,5) | 49 (92,5) | 1 (2,4) | 41 (97,6) |
| Douleurs musculaires | 21 (39,6) | 32 (60,4) | 2 (3,8) | 51 (96,2) | - | 42 (100,0) |
| Céphalée | 30 (56,6) | 23 (43,4) | 8 (15,1) | 45 (84,9) | 1 (2,4) | 41 (97,6) |
| Rigidité | 21 (39,6) | 32 (60,4) | 2 (3,8) | 51 (96,2) | - | 42 (100,0) |

**Tableau 2 - Evaluation de symptômes : Guérison**

| | Visite | | | |
|---|---|---|---|---|
| | Jour 5 (N=53) | | Jour 10 (N=42) | |
| | n | % | n | % |
| ***Guérison*** | | | | |
| Guéris | 8 | 15,1 | 33 | 78,6 |
| En train de guérir | 39 | 73,6 | 7 | 16,7 |
| Inchangés | 3 | 5,7 | 1 | 2,4 |
| Complications | 3 | 5,7 | 1 | 2,4 |

## Revendications

1. Composition pharmaceutique sous forme d'aérosol consistant en un mélange d'huile essentielle de menthe poivrée, un mélange d'excipients, un gaz propulseur liquéfié, un arôme et un édulcorant.

2. Composition pharmaceutique selon la revendication 1, **caractérisé en ce que** le mélange d'excipients est un mélange d'éthanol, de myristate d'isopropyle et/ou de diméthylisosorbide.

3. Composition pharmaceutique selon l'une quelconque des revendications 1 à 2, **caractérisé en ce que** le gaz propulseur est le 1,1,1,2-tétrafluoroéthane (HFA134a) encore appelé norflurane.

4. Composition pharmaceutique selon l'une quelconque des revendications 1 à 3, **caractérisé en ce que** l'édulcorant est la saccharine.

5. Utilisation d'une composition pharmaceutique selon l'un quelconque des revendications 1 à 4 pour la fabrication d'un médicament utiles dans le traitement des états congestifs des voies aériennes supérieures, en cas de nez bouché, de rhume, et comme antalgique dans les affections de la cavité buccale et /ou du pharynx.

6. Procédé de préparation d'une composition pharmaceutique selon l'un quelconque des revendications 1 à 4 qui consiste mélanger le principe actif, le mélange d'excipients et la composition aromatique et enfin à dissoudre l'édulcorant puis à rajouter le gaz propulseur liquéfié.

7. Un aérosol équipé d'une valve distributrice doseuse contenant une composition selon l'une quelconque des revendications 1 à 4 permettant l'administration d'huile essentielle de menthe poivrée à un patient par voie orale ou nasale.

8. Utilisation de l'aérosol selon la revendication 5 pour le traitement des états congestifs des voies aériennes supérieures, en cas de nez bouché, de rhume, et comme antalgique dans les affections de la cavité buccale et /ou du pharynx.

9. Procédé de préparation de l'aérosol selon la revendication 5 qui consiste (a) préparer la composition pharmaceutique selon l'une quelconque des revendications 1 à 4 en mélangeant le principe actif, le mélange d'excipients et la composition aromatique et enfin à dissoudre l'édulcorant puis (b) à remplir un l'aérosol avec la composition pharmaceutique de l'étape (a), (c) à équiper l'aérosol d'une valve distributrice doseuse puis (d) à rajouter le gaz propulseur liquéfié.
